# EUROPÄISCHE PATENTSCHRIFT

(11) **EP 0 990 892 B1**
(45) Veröffentlichungstag und Bekanntmachung des Hinweises auf die Patenterteilung: **12.12.2007**
(21) Anmeldenummer: 99203139.3
(22) Anmeldetag: 24.09.1999
(51) Int. Cl.: G01N 23/04

(54) **Computertomographie-Verfahren mit kegelförmigen Strahlenbündel, und Computertomograph**
CT-Method using a conical beam, and CT Apparatus
Méthode de tomographie assistée par ordinateur utilisant un faisceau cônique, et dispositif de tomographie

(30) Priorität: 01.10.1998 DE 19845133
(43) Veröffentlichungstag der Anmeldung: 05.04.2000
(73) Patentinhaber: Philips Intellectual Property & Standards GmbH, 20099 Hamburg (DE); Koninklijke Philips Electronics N.V., 5621 BA Eindhoven (NL)
(72) Erfinder: Proksa, Roland Philips Corp. Intell. Prop. GMBH, 52064 Aachen (DE); Grass, Michael,Dr. Philips Corp.Intell.Prop.GmbH, 52064 Aachen (DE)
(74) Vertreter: Volmer, Georg

(56) Entgegenhaltungen:
- WO-A-98/30980
- WO-A-99/36885
- US-A- 5 406 479

## Beschreibung

Die Erfindung betrifft ein Computertomographie-Verfahren mit den Schritten
- Erzeugen eines kegelförmigen, einen Untersuchungsbereich bzw. ein darin befindliches Objekt durchsetzenden Strahlenbündels mit einer Strahlenquelle,
- Erzeugung einer kreisförmigen Relativbewegung um eine Rotationsachse zwischen der Strahlenquelle einerseits und dem Untersuchungsbereich bzw. dem Objekt andrerseits,
- Akquisition von Meßdaten, die von der Intensität in dem Strahlenbündel jenseits des Untersuchungsbereiches abhängen, mit einer Detektoreinheit während der Relativbewegung,
- Rekonstruktion der räumlichen Verteilung der Absorption innerhalb des Untersuchungsbereiches aus den akquirierten Meßdaten.

Außerdem bezieht sich die Erfindung auf einen Computertomographen zur Durchführung dieses Verfahrens.

Ein solches Verfahren ist aus dem Aufsatz von L.A. Feldkamp et al "Practical cone-beam algorithm", Journal of Optical Soc. Am.A/Vol.1,No 6,1984, pp. 612-619 bekannt. Das bekannte Verfahren besteht im Prinzip aus den folgenden Schritten:
a) Für jede Strahlenquellenposition werden alle Meßwerte mit einem Gewichtungsfaktor multipliziert, der dem Kosinus des Winkels entspricht, den der Strahl längs dessen der Meßwert erfaßt wurde, mit dem Zentralstrahl einschließt.
b) Die so gewichteten Meßwerte werden einer Hochpaßfilterung unterzogen.
c) Die Meßwerte werden entlang der Strahlen, längs derer sie gemessen worden sind in den Untersuchungsbereich rückprojiziert. Der Beitrag eines Meßwerts zu dem Absorptionswert eines Voxels muß dabei mit einem Faktor gewichtet werden, der vom Abstand des betreffenden Voxels von der Strahlenquellenposition abhängt.

Da dieser letzte Schritt für sämtliche Voxel des zu rekonstruierenden Volumens und für sämtliche Strahlenquellenpositionen durchgeführt werden muß, benötigt er viel Rechenzeit. Ein weiterer Nachteil des bekannten Verfahrens ist, daß man damit die Absorption nur in denjenigen Voxeln rekonstruieren kann, die während der Untersuchung ununterbrochen von Röntgenstrahlung getroffen worden sind. Diese Voxel liegen in einem zur Rotationsachse konzentrischen diskusförmigen Bereich. Erwünscht ist jedoch die Rekonstruktion in einem ebenen scheibenförmigen Bereich. Beschränkt man aber die Rekonstruktion auf den scheibenförmigen Bereich in dem kegelförmigen Strahlenbündel, der aus allen Strahlenquellenpositionen von Röntgenstrahlung getroffen wird, dann ergibt sich lediglich ein sehr schmaler Rekonstruktionsbereich.

Aufgabe der vorliegenden Erfindung ist es ein Computertomographie-Verfahren der eingangs genannten Art so auszugestalten, daß der Rechenaufwand verringert wird und die Rekonstruktion der Absorptionsverteilung in einer dickeren ebenen Scheibe des Untersuchungsbereichs möglich wird. Diese Aufgabe wird ausgehend von einem Verfahren der eingangs genannten Art erfindungsgemäß durch die folgenden Schritte gelöst:
a) Rebinning der Meßdaten zu einer Anzahl von Gruppen, wobei jede Gruppe mehrere zueinander und zur Rotationsachse parallele Ebenen umfaßt, in denen sich je ein Strahlenfächer befindet,
b) Eindimensionale Filterung der durch das Rebinning erzeugten Daten einer jeden Gruppe in Richtung senkrecht zur Richtung der Ebenen,
c) Rekonstruktion der räumlichen Verteilung der Absorption durch Rückprojektion der gefilterten Daten von mehreren Gruppen.

Während bei dem bekannten Verfahren die in den einzelnen Strahlenquellenpositionen gewonnenen Meßwerte jeweils unmittelbar einer Filterung unterzogen werden, erfolgt bei dem erfindungsgemäßen Verfahren zunächst ein Rebinning, bei dem in unterschiedlichen Strahlenquellenpositionen erfaßte Strahlenfächer gruppenweise zusammengefaßt und erst danach gefiltert werden. Der Vorteil des zusätzlichen Rebinning besteht darin, daß bei der anschließenden Rekonstruktion der Absorptionsverteilung die Beiträge der einzelnen Meßwerte zu einem Voxel nicht mit einem abstandsabhängigen Faktor multipliziert werden müssen. Dadurch vereinfacht sich das Rekonstruktionsverfahren ganz deutlich. Es ergibt sich auch eine verbesserte Bildqualität. Außerdem ergibt sich ein günstiges Verhältnis zwischen dem bestrahlten Volumen und dem Volumen, innerhalb dessen die Rabsorptionsverteilung rekonstruiert werden kann.

Die Erfindung geht weiter von der Erkenntnis aus, daß sich die Absorption in jedem Voxel rekonstruieren läßt, wenn es über einen Winkelbereich von wenigstens 180° bestrahlt worden ist. Es läßt sich zeigen, daß alle Voxel, die diese Bedingung erfüllen, in einer ebenen, zur Rotationsachse senkrechten Scheibe liegen, deren Dicke größer ist als die Abmessungen an den Rändern des diskusförmigen Bereichs, in dem die Absorption mit dem bekannten Verfahren rekonstruiert werden kann.

Das in Anspruch 2 vorgesehene Rebinning auf einen ebenen und rechteckigen virtuellen Detektor erleichtert die nachfolgende eindimensionale Filterung ganz wesentlich.

In der Scheibe, in der die Absorptionsverteilung rekonstruiert werden kann, befinden sich Voxel, die aus einem Winkelbereich von mehr als 180° bestrahlt worden sind. Da für die Rekonstruktion aber nur ein Bestrahlungswinkelbereich (das ist der Winkelbereich, den die (Parallel-) Projektion der Strahlen von der Strahlenquelle zu einem Voxel auf eine zur Rotationsachse senkrechte Ebene in dieser Ebene bedecken, bzw. den die Komponenten der Vektoren von der Strahlenquelle zu demVoxel in der Rotationebene der Strahlenquelle überstreichen) von 180° erforderlich ist, können Meßwerte aus einigen Strahlenquellenpositionen unberücksichtigt bleiben. Anspruch 3 beschreibt nun eine einfache Möglichkeit, wie man die Strahlenquellenpositionen ermitteln kann, deren Meßdaten für die Rekonstruktion der Absorptionen in einzelnen Voxeln heranzuziehen sind.

Grundsätzlich kann man die Absorptionsverteilung in der erwähnten Scheibe rekonstruieren, indem man für alle Voxel nur so viele Strahlenquellenpositionen zur Rekonstruktion heranzieht, daß sich gerade ein Bestrahlungswinkelbereich von 180° ergibt. Es gibt in dieser Scheibe jedoch auch Voxel mit einem Bestrahlungswinkelbereich von 360°. Wenn man diese Voxel ebenfalls nur mit einem Bestrahlungswinkelbereich von 180° rekonstruieren würde, würde sich für diese Voxel nicht das bestmögliche Signal/Rauschverhältnis ergeben. Anspruch 4 beschreibt daher ein hybrides Rekonstruktionsverfahren, bei dem das Volumen der rekonstruierbaren Scheibe in zwei Teilvolumina unterteilt wird: in ein erstes Teilvolumen., das nur Voxel umfaßt, die aus allen Strahlenquellenpositionen bestrahlt worden sind und in ein zweites Teilvolumen, bei dem der Bestrahlungswinkelbereich kleiner ist als 360°. Bei der Rekonstruktion der Absorption in den Voxeln des ersten Teilvolumens werden sämtliche Meßwerte herangezogen und bei der Rekonstruktion der Voxel in dem zweiten Teilvolumen nur die Meßwerte aus einem Bestrahlungswinkelbereich von 180°.

Anspruch 5 beschreibt einen Computertomographen zur Durchführung des erfindungsgemäßen Verfahrens.

Anspruch 6 beschreibt einen Computertomographen im Zusammenhang mit einer Ausgestaltung des erfindungsgemäßen Verfahrens, die darauf abstellt, daß ein Teil der Meßwerte für die Rekonstruktion nicht herangezogen wird. Diese Meßwerte gehören zu Strahlen, die nicht benötigt werden, wenn man nur die Absorptionsverteilung in einer ebenen Schicht rekonstruieren will. Diese Strahlen haben in einer zur Rotationsachse senkrechten Ebene einen kleinen Öffnungswinkel und in einer die Rotationsachse enthaltenden Ebene einen relativ großen Öffnungswinkel. Wenn man das kegelförmige Strahlenbündel durch die in Anspruch 6 angegebene Gestaltung der Kollimatoranordnung so ausformt, daß diese Strahlen gar nicht erst entstehen, wird die Strahlenbelastung für einen im Untersuchungsbereich befindlichen Patienten verringert. Dieser Vorteil ergibt sich auch dann wenn man die Absorptionsverteilung in anderer Weise rekonstruiert, z.B. durch ein ART-Verfahren.

Die Erfindung wird nachstehend anhand der Zeichnungen näher erläutert. Es zeigen:
- Fig. 1: Einen Computertomographen mit dem die Erfindung ausführbar ist.
- Fig. 2: Ein Ablaufdiagramm für das erfindungsgemäße Rekonstruktionsverfahren.
- Fig. 3: Ein in einer Strahlenquellenposition erzeugtes kegelförmiges Strahlenbündel.
- Fig. 4: Die durch das Rebinning gebildeten Strahlenfächer in parallelen Ebenen.
- Fig. 5: Einen Querschnitt durch diesen Strahlenfächer.
- Fig. 6: Die geometrischen Verhältnisse im Untersuchungsbereich.
- Fig. 7: Ein Diagramm zur Auswahl von Strahlenquellenpositionen.

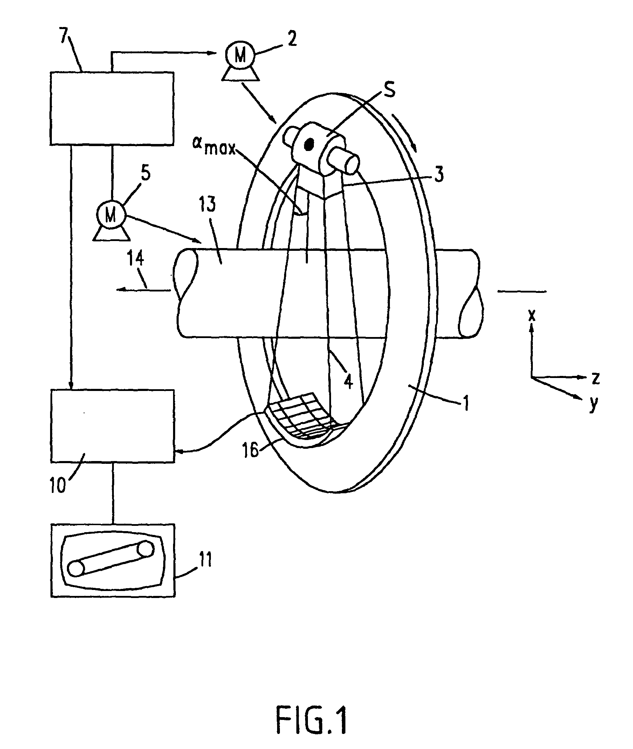

Der in Fig. 1 dargestellte Computertomograph umfaßt eine Gantry 1, die um eine parallel zur z-Richtung, des in Fig. 1 dargestellten Koordinatensystems verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S, beispielsweise ein Röntgenstrahler, befestigt. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in z-Richtung als auch in einer dazu senkrechten Richtung (d.h. in einer zur Rotationsachse senkrechten Ebene) eine von Null verschiedene, endliche Ausdehnung hat.

Das Strahlenbündel 4 durchdringt einen Untersuchungsbereich 13, in dem sich ein Patient auf einem Patientenlagerungstisch (beides nicht näher dargestellt) befinden kann. Der Untersuchungsbereich 13 hat die Form eines Zylinders, der im folgenden als Objektzylinder 13 bezeichnet wird. Nach dem Durchsetzen des Objektzylinders 13 trifft das Röntgenstrahlenbündel 4 auf eine an der Gantry 1 befestigte zweidimensionale Detektoreinheit 16, die eine Anzahl von Detektorzeilen mit jeweils einer Vielzahl von Detektorelementen umfaßt. Die Detektorzeilen befinden sich in zur Rotationsachse senkrechten Ebenen auf einem Kreisbogen um die Strahlenquelle S; sie können aber auch einen Kreisbogen um die Rotationsachse beschreiben oder geradlinig sein. Jedes Detektorelement liefert in jeder Strahlenquellenposition einen Meßwert für einen Strahl aus dem Strahlenbündel 4.

Der mit αₘₐₓ bezeichnete Öffnungswinkel des Strahlenbündels 4 (als Öffnungswinkel ist der Winkel definiert, den ein Strahl den einer zur Rotationsachse 14 senkrechten Ebene am Rande des Strahlenbündels 4 liegt, mit einer durch die Strahlenquelle S und die Rotationsachse 14 definierten Zentralstrahlebene einschließt) bestimmt dabei den Durchmesser des Objektzylinders 13, innerhalb dessen sich das zu untersuchende Objekt bei der Akquisition der Meßwerte befindet. Der Untersuchungsbereich 13 - bzw. das Objekt oder der Patientenlagerungstisch - kann mittels eines Motors 5 parallel zur Rotationsachse 14 bzw. zur z-Achse verschoben werden. Die Geschwindigkeit dieses Vorschubs in z-Richtung ist vorzugsweise konstant und einstellbar. Wenn die Motoren 5 und 2 gleichzeitig laufen, ergibt sich eine helixförmige Abtastbewegung der Strahlenquelle S und der Detektoreinheit. Wenn hingegen der Motor 5 für den Vorschub in z-Richtung stillsteht und der Motor 2 die Gantry einzeln rotieren läßt, ergibt sich eine kreisförmige Abtastbewegung der Strahlenquelle S und der Detektoreinheit relativ zum Untersuchungsbereich 13. Im folgenden soll nur diese kreisförmige Abtastbewegung betrachtet werden.

Die von der Detektoreinheit akquirierten Meßdaten werden einem Bildverarbeitungsrechner 10 zugeführt, der daraus die Absorptionsverteilung in einem Teil des Untersuchungsbereichs 13 rekonstruiert und z.B. auf einem Monitor 11 wiedergibt. Die beiden Motoren 2 und 5, der Bildverarbeitungsrechner 10, die Strahlenquelle S und der Transfer der Meßdaten von der Detektoreinheit 16 zum Bildverarbeitungsrechner 10 werden von einer geeigneten Kontrolleinheit gesteuert.

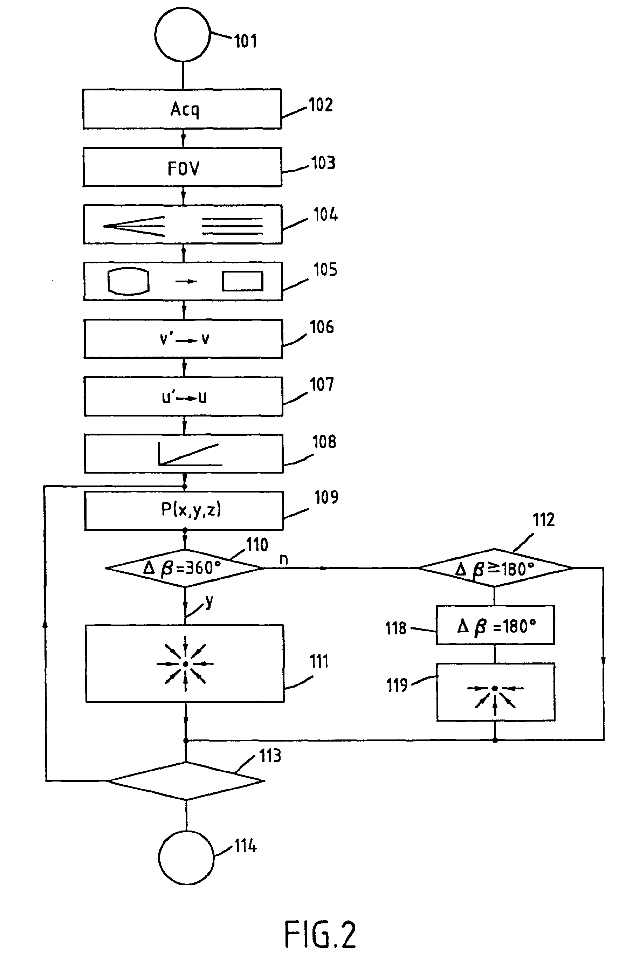

Fig. 2 zeigt den Ablauf eines Meß- und Rekonstruktionsverfahrens, das mit dem Computertomographen nach Fig. 1 durchgeführt werden kann.

Nach der Initialisierung im Block 101 rotiert die Gantry mit einer konstanten Winkelgeschwindigkeit. Im Schritt 102 wird die Strahlung der Strahlenquelle S eingeschaltet, und die dabei von den Detektorelementen der Detektoreinheit 16 akquirierten Meßwerte werden in einem Speicher des Bildverarbeitungsrechners 10 gespeichert.

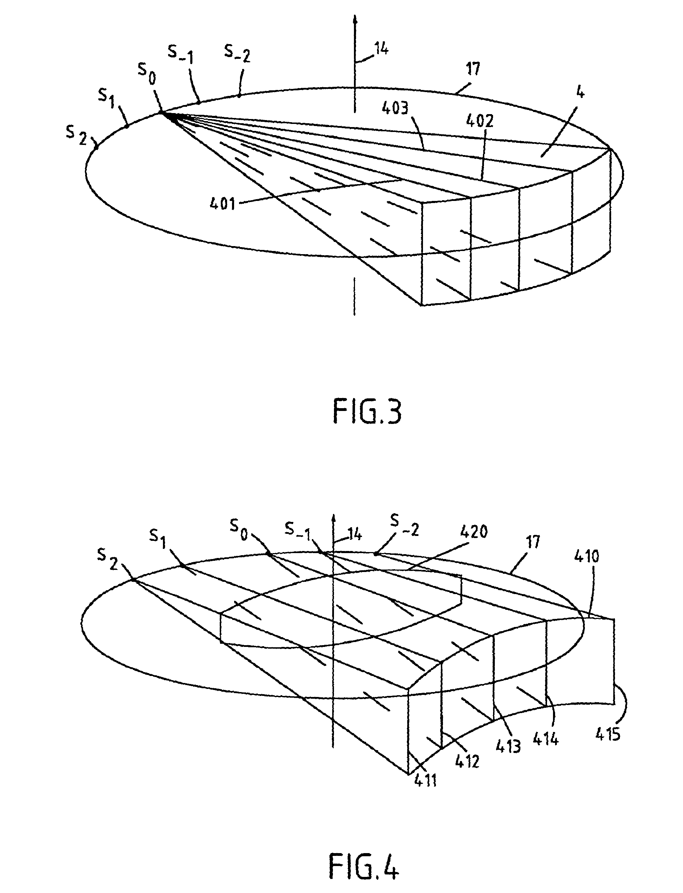

Fig. 3 zeigt die kreisförmige Bahn 17, auf der sich die Strahlenquelle S und die Detektoreinheit 16 um die Rotationsachse 14 bewegen. Für eine bestimmte Strahlenquellenposition S₀ ist das Strahlenbündel 4 dargestellt. Man kann sich dieses kegelförmige Strahlenbündel 4 aus einer Vielzahl von ebenen Strahlenfächern zusammensetzen, die sich - wie die Strahlenfächer 401 ...403 in zur Rotationsachse parallelen Ebenen befinden. Obwohl man die Strahlen des kegelförmigen Strahlenbündels 4 auch auf andere Weise zusammenfassen kann, soll der Begriff "Strahlenfächer" im folgenden nur für solche Strahlen verwendet werden, die in einer gemeinsamen, zur Rotationsachse 14 parallelen Ebene liegen. Diese Strahlenfächer gehen von einer Strahlenquellenposition aus und werden von je einer zur Rotationsrichtung parallelen Spalte von Detektorelementen auf der Detektoreinheit 16 erfaßt. Fig. 3 deutet an, daß auch in anderen Positionen der Strahlenquelle (z.B. S₋₁, S₁ oder S₂) das emittierte kegelförmige Strahlenbündel gemessen wird.

Im Schritt 103 wird der Durchmesser des darzustellenden Bereichs FOV (field of view) festgelegt. Der Durchmesser kann dem durch αₘₐₓ definierten Durchmesser des Untersuchungsbereichs 13 entsprechen; er kann jedoch auch kleiner sein.

In den Schritten 104-107 erfolgt ein Rebinning. Die Daten werden dabei so umsortiert und uminterpoliert, als wären Sie mit einer anderen Strahlenquelle (einer kreisbogenförmigen Strahlenquelle, die zueinander parallele Strahlenfächer emittiert) und mit einem anderen Detektor (einem ebenen, rechteckigen und die Rotationsachse enthaltenden "virtuellen" Detektor) gemessen worden.

Im Schritt 104 werden dazu zunächst die Strahlenfächer von verschiedenen Strahlenquellenpositionen zu jeweils einer Gruppe zusammengefaßt, die in zueinander parallelen Ebenen liegen. Die zu einer Gruppe gehörenden Strahlenfächer erfüllen daher die Bedingung, daß die Summe der Winkel α und β für alle Strahlenfächer dieser Gruppe den gleichen Wert hat. Dabei ist α der Winkel, den die Ebene des Strahlenfächers mit einer durch die Strahlenquellenposition und die Rotationsachse definierten Zentralstrahlebene einschließt und der durch die Lage der Spalte von Detektorelementen gegeben ist, die den betreffenden Strahlenfächer erfaßt hat. β ist ein die Strahlenquellenposition (z.B. S₀) auf dem Kreis 17 kennzeichnender Winkel. Wenn die Strahlenfächer für eine Strahlenquellenposition diese Bedingung nicht exakt erfüllen, muß für diese Strahlenquellenposition ein entsprechender Strahlenfächer durch Interpolation aus den Strahlen benachbarter Strahlenfächer ermittelt werden.

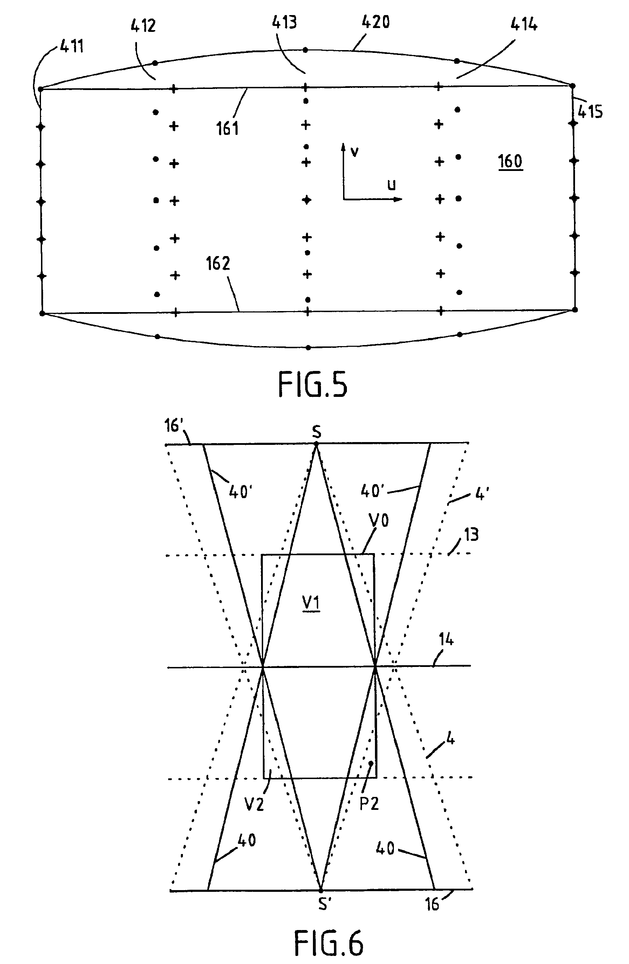

Fig. 4 zeigt eine auf diese Weise gebildete Gruppe von Strahlenfächern. Von jeder der einander benachbarten Strahlenquellenpositionen S₋₂....S₀...S₂ gehört jeweils ein Strahlenfächer zu einer Gruppe. Jede Gruppe kann durch den Winkel β der Strahlenquellenposition (z.B. S₀) gekennzeichnet werden, deren zu der Gruppe gehörender Strahlenfächer die Rotationsachse 14 durchsetzt. (In der Regel ist dies die mittlere Strahlenquellenposition - im Beispiel nach Fig. 4 also die Strahlenquellenposition S₀). - Es können sich dann so viele Gruppen von Strahlenfächern ergeben , wie es Strahlenquellenpositionen gibt, aber auch mehr oder weniger.

Die auf diese Weise bestimmten Strahlenfächer - darunter die in Fig. 4 dargestellten Strahlenfächer 411...415 - definieren ein Strahlenbündel 410, das eine zeltartige Form hat und sich aus Strahlenfächern zusammensetzt, die in zueinander und zur Rotationsachse parallelen Ebenen liegen. In Fig. 4 ist außerdem der Schnittbereich 420 dargestellt, der sich ergibt, wenn das Strahlenbündel 410 von einer die Rotationsachse 14 enthaltenden und zu den Ebenen der Strahlenfächer 411...415 senkrechten Ebene geschnitten wird. Wie insbesondere Fig. 5 zeigt, die diesen Schnittbereich darstellt, sind die oberen und unteren Ränder gewölbt. Diese Wölbung ist darauf zurückzuführen, daß die Strahlenquellen positionen in der Mitte (z.B. S₀) weiter von der Schnittebene entfernt sind als die am Rande S₂ oder S₋₂ und daß die Fächer alle den gleichen Öffnungswinkel haben, weil die Detektorzeilen einen Kreisbogen um die Strahlenquelle S beschreiben. Bei einer anderen Geometrie der Detektorzeilen würde sich die Form der Schnittebene 420 ändern. Bei einer ebenen Detektoreinheit (z.B. mit gradlinigen Detektorzeilen) wäre die Wölbung noch ausgeprägter, weil die am Rande liegenden Strahlenfächer , wie z.B. 411 und 415, dabei einen geringeren Öffnungswinkel hätten.

Für jede Gruppe von Strahlenfächern wird daher in dem ebenen Schnittbereich 420 ein rechteckiger virtueller Detektor definiert (Schritt 105), dessen oberer und unterer Rand 161 bzw. 162 durch die Abmessungen der äußeren Strahlenfächer 411 bzw. 415 in den ebenen Schnittbereich gegeben sind. Wenn der Durchmesser des zu rekonstruierenden FOV (field of view) kleiner gewählt worden ist als der Durchmesser des Untersuchungsbereichs 13, können von jeder Gruppe jeweils die äußeren Strahlenfächer (z.B. 411, 415) entfallen. Der Abstand der oberen und unteren Ränder 161 und 162 kann in diesem Fall größer gewählt werden als bei dem in Fig. 5 dargestellten Beispiel.

In Fig. 5 sind außerdem - durch runde Punkte markiert - die Durchstoßpunkte einiger in den Strahlenfächern 411---415 enthaltener Strahlen durch diesen virtuellen Detektor dargestellt. Schließlich sind durch Kreuze die Stützpunkte eines regelmäßigen kartesischen Gitters angedeutet. Die Durchstoßpunkte und die Stützpunkte fallen durchweg nicht zusammen. Man erkennt einerseits, daß die Strahlenfächer außen enger beieinanderliegen als innen und daß die Durchstoßpunkte eines Strahlenfächers innen weiter voneinander entfernt sind als außen. Aus den Meßwerten für die Durchstoßpunkte müssen daher in den beiden folgenden Schritten 106 und 107 die Meßwerte an den äquidistanten Stützstellen innerhalb des virtuellen Detektors 160 ermittelt werden.

Im Schritt 106 erfolgt dabei zunächst eine vertikale Interpolation derart, daß die Stützstellen in vertikaler Richtung für alle Strahlenfächer voneinander den gleichen Abstand haben, wie die Durchstoßpunkte bzw. Stützstellen am Rande des virtuellen Detektors 160. Im Schritt 107 erfolgt eine Interpolation in horizontaler Richtung, so daß man interpolierte Werte für Stützstellen erhält, die innerhalb des virtuellen Detektors 160 in horizontaler Richtung voneinander den gleichen Abstand haben. Die Interpolationsschritte 105 und 106 können auch miteinander vertauscht und ggf. sogar miteinander kombiniert werden.

Das Ergebnis dieser Interpolation in horizontaler und vertikaler Richtung ist, daß innerhalb des virtuellen Detektors 160 die Intensität der Strahlung an den äquidistanten Stützpunkten eines regelmäßigen kartesischen Gitters vorliegt. Die Stützstellen definieren ihrerseits wiederum neue Strahlenfächer, die in parallelen Ebenen mit jeweils dem gleichen Abstand voneinander liegen. Nur diese neuen Strahlenfächer (die teilweise mit den originalen Strahlenfächern identisch sein können) werden für die weitere Rekonstruktion benutzt.

Ein Teil der Strahlen, deren Durchstoßpunkte außerhalb des virtuellen Fensters liegen, werden in den Schritten 106 und 107 nicht benutzt. Es ist daher von Vorteil, den Kollimator 3 von vornherein so zu gestalten, daß das kegelförmige Strahlenbündel von vornherein nicht enthält. Dadurch würde die Strahlenbelastung für den Patienten verringert. Anstelle von geradlinigen senkrecht zur Rotationsachse verlaufenden Kanten müßte die Kollimatoranordnung zu diesem Zweck nach innen gewölbte Kanten aufweisen, so daß die Strahlenfächer, die die Rotationsachse schneiden oder davon einen geringen Abstand haben, einen geringeren Öffnungswinkel hätten, als Strahlenfächer am äußeren Rand des Strahlenbündels.

Nach den Schritten 103 bis 107 ist somit für jede Gruppe von Strahlenfächern die Strahlenintensität an den regelmäßigen Stützstellen des zu dieser Gruppe gehörenden virtuellen Detektors 160 bestimmt. Dies erleichtert die erforderliche Hochpaßfilterung fundamental, weil lediglich eine eindimensionale Filterung der durch das Rebinning auf den virtuellen Detektor 160 erzeugten Daten notwendig ist. Im Schritt 108 wird daher auf diese Daten eine eindimensionale Filterung mit einem rampenförmig mit der Frequenz ansteigenden Übertragungsfaktor angewandt. Dazu werden jeweils lediglich die Werte von in horizontaler Richtung aufeinander folgenden Stützstellen herangezogen. Diese Filterung wird für alle Gruppen von (neuen) Strahlenfächern ausgeführt.

Die nach dem Rebinning und der Filterung für die durch die Stützstellen im virtuellen Fenster 160 definierten Strahlen ermittelten Daten werden anschließend zur Rekonstruktion der Absorptionsverteilung im Untersuchungsbereich durch eine Rückprojektion herangezogen. Durch das vorangegangenen Schritte 103-108 ist es dabei aber - im Gegensatz zu dem eingangs erwähnten bekannten Verfahren - nicht erforderlich, die Beiträge einzelner Strahlen zu dem Absorptionswert eines Voxels mit einem Faktor zu gewichten werden, der vom Abstand des betreffenden Voxels von der Strahlenquellenposition abhängt.

Im Prinzip würde es für die Rückprojektion genügen, wenn man für jedes Voxel Daten aus Gruppen heranzieht, deren Strahlenfächer miteinander einen Winkelbereich von insgesamt nur 180° definieren. Es gibt jedoch im Untersuchungsbereich eine Vielzahl von Voxeln, die in sämtlichen Strahlenquellenpositionen von Strahlung getroffen worden sind und es ist empfehlenswert für diese Voxel sämtliche verfügbaren Meßwerte heranzuziehen, um ein möglichst gutes Signal/Rauschverhältnis zu erreichen. Deshalb wird im folgenden danach differenziert, ob die Voxel aus allen Strahlenquellenpositionen bestrahlt worden sind oder nicht.

Nach der Vorgabe eines Voxels im Untersuchungsbereich, dessen Absorption rekonstruiert werden soll (Schritt 109), wird im Schritt 110 zunächst geprüft, ob das betreffende Voxel aus allen Strahlenquellenpositionen bestrahlt worden ist.

Dazu wird zunächst auf Fig. 6 hingewiesen, die die durch einen Punkt angedeutete Strahlenquelle S, die durch eine Linie symbolisierte Detektoreinheit 16 und das Strahlenbündel 4 in einer ersten Position bezüglich der Rotationsachse 14 zeigt. Außerdem zeigt Fig. 6 in einer zweiten, gegenüber der ersten um 180° versetzten Position die Strahlenquelle S', die Detektoreinheit 16' und das Strahlenbündel 4'. Außerdem ist in Fig. 6 der Untersuchungsbereich 13 angedeutet. Die Strahlen an den in Richtung der Rotationsachse 14 gegeneinander versetzten Rändern des Strahlenbündels 4 sind gestrichelt angedeutet. Mit ausgezogenen Linien sind hingegen die Strahlen 40 bzw. 40' angedeutet, die nach dem Weglassen der Strahlen am Rande (Schritt 107) das Strahlenbündel begrenzen.

Es läßt sich zeigen, daß alle Voxel innerhalb einer ebenen kreisförmigen Scheibe V₀ aus einem Winkelbereich von zumindest 180° bestrahlt worden sind. Die Seitenflächen dieser Scheibe sind durch die Schnittpunkte der Randstrahlen 40 bzw. 40' mit der Rotationsachse 14 definiert. Alle Voxel, die innerhalb des durch die Randstrahlen 40 bzw. 40' definierten, diskusförmigen Teilvolumens V₁ liegen, sind dabei aus sämtlichen Strahlenquellenpositionen bestrahlt worden, während die Voxel, die in dem durch die Differenz von V₀ und V₁ gegebenen Teilvolumen V₂ liegen, die Strahlenquelle bei der Abtastbewegung aus einem Winkel von mindestens 180° aber weniger als 360° "gesehen" haben.

Ergibt nun die Abfrage 110, daß das im Schritt 109 ausgewählte Voxel im Teilvolumen V₁ liegt, daß es also aus allen Strahlenquellenpositionen bestrahlt worden ist, dann erfolgt im Schritt 111 eine Rückprojektion, wobei von allen Gruppen jeweils derjenige Strahl in dieser Gruppe herangezogen wird, der durch das betreffende Voxel verläuft. Passiert kein Strahl exakt die Mitte dieses Voxels, dann muß der zugehörige Wert durch Interpolation der Meßwerte von benachbarten Strahlen ermittelt werden.

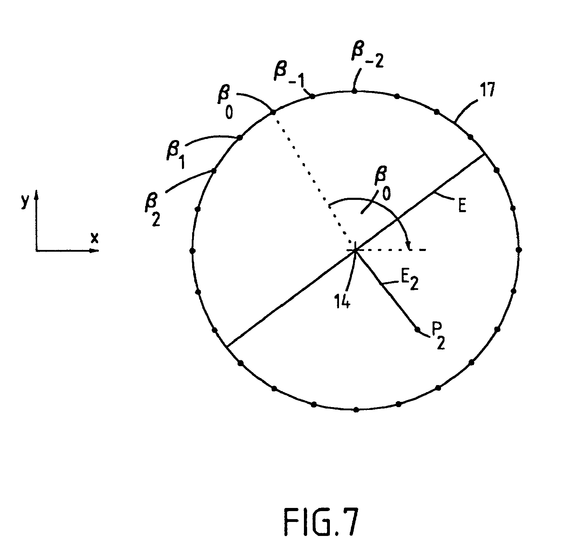

Ist im Schritt 110 die Abfrage negativ, dann erfolgt im Schritt 112 eine weitere Abfrage, ob das ausgewählte Voxel im Teilvolumen V₂ liegt (ob es also die Strahlenquelle unter einem Winkel von wenigstens 180° "gesehen" hat) ist dies nicht der Fall, dann verzweigt das Programm zu einer weiteren Abfrage 113, in der festgestellt wird, ob die Absorption schon in sämtlichen Voxeln des FOV rekonstruiert worden ist. Ist dies nicht der Fall, dann werden die Schritte 109 ff erneut durchlaufen. Bei einer positiven Antwort, d.h. wenn sich das im Schritt 109 ausgewählte Voxel in dem Volumen V₂ befindet, werden im Schritt 118 die Gruppen ausgewählt, die zur Rekonstruktion der Absorption in dem Voxel herangezogen werden. Dazu wird auf Fig. 7 verwiesen, die die kreisförmige Abtastbahn 17 zeigt und auf ihr - durch Punkte angedeutet - die verschiedenen Strahlenquellenpositionen. Die in Fig. 4 mit S₋₂ ...S₀...S₂ bezeichneten Strahlenquellenpositionen sind in Fig. 7 durch die zugehörigen Winkel β₋₂...β₀...β₂ auf dem Kreis 17 gekennzeichnet. Wie in Verbindung mit den Schritten 103 bis 108 erläutert, ist jeder Strahlenquellenposition eine Gruppe von durch das Rebinning entstandenen gefilterten Meßdaten zugeordnet.

Außerdem ist in Fig. 7 ein Voxel P₂ dargestellt, das - wie in Fig. 6 angedeutet - in dem Teilvolumen V₂ liegt. Mit E₂ ist dabei die zur Zeichenebene der Fig. 7 senkrechte Ebene bezeichnet, die durch das Voxel P₂ und die Rotationsachse 14 definiert wird. Schließlich ist mit E eine Ebene bezeichnet, die die Ebene E₂ in der Rotationsachse schneidet.

Es läßt sich zeigen, daß das Voxel P₂ aus allen Strahlenquellenpositionen jenseits der Ebene E bestrahlt worden ist. Hingegen hat das Voxel P₂ allenfalls von einigen - nicht aber von allen - diesseits der Ebene E (in bezug auf das Voxel) befindlichen Strahlenquellenpositionen Strahlung empfangen.

Infolgedessen werden im Schritt 119 alle durch das Rebinning und das Filtern entstandenen Gruppen von Daten zur Rekonstruktion herangezogen, die den jenseits der Ebene E befindlichen Strahlenquellenpositionen (z.B. β₂...β₋₂) zugeordnet sind und die miteinander einen Bestrahlungswinkelbereich von genau 180° bilden. Dieser Bestrahlungswinkelbereich ist notwendig und hinreichend, um die Absorption in dem betreffenden Voxel zu rekonstruieren, auch wenn die mit diesen Daten durchgeführte Rückprojektion ein schlechteres Signal/Rauschverhältnis ergibt als die im Schritt 111 durchgeführte Rückprojektion.

Sobald im Schritt 113 festgestellt ist, daß alle Voxel in dem ausgewählten FOV rekonstruiert worden sind, ist das Verfahren damit beendet (Schritt 114). Die Absorptionswerte in dem FOV können dann - ggf. scheibenweise - auf einem geeigneten Display wiedergegeben werden.

## Patentansprüche

1. Computertomographie-Verfahren mit den Schritten
- Erzeugen eines kegelförmigen, einen Untersuchungsbereich bzw. ein darin befindliches Objekt durchsetzenden Strahlenbündels mit einer Strahlenquelle,
- Erzeugung einer kreisförmigen Relativbewegung um eine Rotationsachse zwischen der Strahlenquelle einerseits und dem Untersuchungsbereich bzw. dem Objekt andrerseits,
- Akquisition von Meßdaten, die von der Intensität in dem Strahlenbündel jenseits des Untersuchungsbereiches abhängen, mit einer Detektoreinheit während der Relativbewegung,
- Rekonstruktion der räumlichen Verteilung der Absorption innerhalb des Untersuchungsbereiches aus den akquirierten Meßdaten
**gekennzeichnet durch** folgende Schritte:
a) Rebinning der Meßdaten zu einer Anzahl von Gruppen, wobei jede Gruppe mehrere zueinander und zur Rotationsachse parallele Ebenen umfaßt, in denen sich je ein Strahlenfächer befindet,
b) Eindimensionale Filterung der **durch** das Rebinning erzeugten Daten einer jeden Gruppe in Richtung senkrecht zur Richtung der Ebenen,
c) Rekonstruktion der räumlichen Verteilung der Absorption **durch** Rückprojektion der gefilterten Daten von mehreren Gruppen.

2. Computertomographie-Verfahren nach Anspruch 1,
**dadurch gekennzeichnet, daß** das Rebinning auf je einen zu den Ebenen einer jeden Gruppe senkrechten, ebenen und rechteckigen virtuellen Detektor erfolgt, der die Rotationsachse enthält.

3. Computertomographie-Verfahren nach Anspruch 1,
**gekennzeichnet durch** die Rekonstruktion der Absorption der Voxel mit einem Bestrahlungswinkelbereich von mindestens 180° unter Heranziehung der gefilterten Meßdaten von ausschließlich denjenigen Strahlenquellenpositionen, die von dem Voxel durch eine die Rotationsachse enthaltende Ebene getrennt sind, welche zu einer **durch** das betreffende Voxel und die Rotationsachse definierten Ebene senkrecht steht.

4. Computertomographie-Verfahren nach Anspruch 1,
**gekennzeichnet durch** folgende Schritte:
a) Zuordnung von Voxeln mit einem Bestrahlungswinkelbereich von 360° zu einem ersten Teilvolumen,
b) Zuordnung von Voxeln mit einem Bestrahlungswinkelbereich von mindestens 180° aber weniger als 360° zu einem zweiten Teilvolumen,
c) Rekonstruktion der Absorption der Voxel in dem ersten Teilvolumen unter Heranziehung von Meßdaten von sämtliche Strahlenquellenpositionen,
d) Rekonstruktion der Absorption der Voxel in dem zweiten Teilvolumen unter Heranziehung der gefilterten Meßdaten von gerade so vielen Strahlenquellenpositionen, , daß sich ein Bestrahlungswinkelbereich von 180° ergibt..

5. Computertomograph, eingerichtet zur Durchführung des Verfahrens nach Anspruchs 1 mit einer Strahlenquelle und einer damit gekoppelten Detektoreinheit sowie mit einer Antriebsanodnung, um ein im Untersuchungsbereich enthaltenes Objekt und die Strahlenquelle relativ zueinander um eine Rotationsachse rotieren zu lassen und mit einer Rekonstruktionseinheit zur Rekonstruktion der räumlichen Verteilung der Absorption innerhalb des Untersuchungsbereiches aus den von der Detektoreinheit akquirierten Meßdaten,
**gekennzeichnet durch**
a) Mittel zum Rebinning der Meßdaten zu mehreren Gruppen, wobei jede Gruppe mehrere zur Rotationsachse parallele Ebenen umfaßt, in denen sich je ein Strahlenfächer befindet,
b) Mittel zur eindimensionalen Filterung der **durch** das Rebinning erzeugten Daten einer jeden Gruppe in Richtung senkrecht zu zur Richtung den Ebenen
c) Mittel zur Rekonstruktion der räumlichen Verteilung der Absorption aus den gefilterten Daten verschiedener Gruppen

6. Computertomograph, nach Anspruch 5, mit einer derart geformten Kollimatoranordnung, dass das kegelförmige Strahlenbündel parallel zur Rotationsachse so geöffnet ist, dass der entsprechende Öffnungswinkel in der Mitte des kegelförmigen Strahlenbündels geringer ist als an den Rändern.

## Claims

1. A computed tomography method which includes the following steps:
- generating a conical radiation beam by means of a radiation source, which beam traverses an examination zone or an object present therein,
- generating a circular relative motion about an axis of rotation between the radiation source on the one side and the examination zone or the object on the other side,
- acquiring, while using a detector unit, measuring data which are dependent on the intensity in the radiation beam at the other side of the examination zone during the relative motion,
- reconstructing the spatial distribution of the absorption within the examination zone from the acquired measuring data,
**characterized by** the following steps:
a) rebinning the measuring data so as to form a number of groups, each group containing a plurality of planes which extend parallel to one another and parallel to the axis of rotation and in each of which a respective fan beam is situated,
b) one-dimensional filtering of the data produced by the rebinning in each group in a direction perpendicular to the direction of the planes,
c) reconstructing the spatial distribution of the absorption by back projection of the filtered data of a plurality of groups.

2. A computed tomography method as claimed in Claim 1,
**characterized in that** the rebinning is performed on a respective flat and rectangular virtual detector which extends perpendicularly to the planes of each group and contains the axis of rotation.

3. A computed tomography method as claimed in Claim 1,
**characterized in that** it includes the reconstruction of the absorption of the voxels with an irradiation angle range of at least 180°, while taking into account the filtered measuring data from exclusively those radiation source positions which are separated from the voxel by a plane containing the axis of rotation and extending perpendicularly to the plane defined by the relevant voxel and the axis of rotation.

4. A computed tomography method as claimed in Claim 1,
**characterized by** the following steps:
a) assigning voxels with an irradiation angle range of 360° to a first sub-volume,
b) assigning voxels with an irradiation angle range of at least 180° but less than 360° to a second sub-volume,
c) reconstructing the absorption of the voxels in the first sub-volume while taking into account measuring data from all radiation source positions,
d) reconstructing the absorption of the voxels in the second sub-volume while taking into account the filtered measuring data from exactly so many radiation source positions that an irradiation angle range of 180° is obtained.

5. A computed tomography apparatus designed for carrying out the method claimed in Claim 1, including a radiation source and a detector unit which is coupled thereto, a drive device for making an object present in the examination zone and the radiation source perform a rotation about the axis of rotation relative to one another, and also including a reconstruction unit for reconstructing the spatial distribution of the absorption within the examination zone from the measuring data acquired by the detector unit,
**characterized by**
a) means for the rebinning of the measuring data into a plurality of groups, each group containing several planes which are parallel to the axis of rotation and each of which contains a fan beam,
b) means for the one-dimensional filtering of the data produced by the rebinning of each group in the direction perpendicular to the direction of the planes,
c) means for the reconstruction of the spatial distribution of the absorption from the filtered data of different groups.

6. A computed tomography apparatus as claimed in Claim 5,
having a collimator device which is shaped such that the conical radiation beam has an aperture parallel to the axis of rotation such that the respective angle of aperture is smaller in the center of the conical radiation beam than at the edges thereof.

## Revendications

1. Procédé de tomographie assistée par ordinateur comprenant les étapes suivantes:
- production d'un faisceau de rayons conique traversant une zone d'examen ou un objet présent dans cette zone d'examen, au moyen d'une source de rayons,
- production d'un déplacement relatif circulaire autour d'un axe de rotation entre la source de rayons, d'une part, et la zone d'examen ou l'objet, d'autre part,
- acquisition de données de mesure qui sont fonction de l'intensité dans le faisceau de rayons au-delà de la zone d'examen, au moyen d'une unité de détecteurs, pendant le déplacement relatif,
- reconstruction de la répartition spatiale de l'absorption à l'intérieur de la zone d'examen à partir des données de mesure acquises,
**caractérisé par** les étapes suivantes:
a) réarrangement des données de mesure en un certain nombre de groupes, chaque groupe comportant plusieurs plans parallèles les uns aux autres et à l'axe de rotation, dans lesquels se trouve chaque fois un éventail de rayons,
b) filtrage monodimensionnel des données produites par le réarrangement de chaque groupe dans une direction perpendiculaire à la direction des plans,
c) reconstruction de la répartition spatiale de l'absorption par rétroprojection des données filtrées de plusieurs groupes.

2. Procédé de tomographie assistée par ordinateur suivant la revendication 1, **caractérisé en ce que** le réarrangement s'effectue sur chaque fois un détecteur virtuel, rectangulaire et plan, perpendiculaire aux plans de chaque groupe, qui contient l'axe de rotation.

3. Procédé de tomographie assistée par ordinateur suivant la revendication 1, **caractérisé par** la reconstruction de l'absorption du voxel avec un domaine d'angle d'irradiation d'au moins 180° eu égard aux données de mesure filtrées provenant exclusivement des positions de sources de rayons, qui sont séparées du voxel par un plan contenant l'axe de rotation, qui est perpendiculaire à un plan défini par le voxel en question et l'axe de rotation.

4. Procédé de tomographie assistée par ordinateur suivant la revendication 1, **caractérisé par** les étapes suivantes:
a) attribution de voxels à domaine d'angle d'irradiation de 360° à un premier volume partiel,
b) attribution de voxels à domaine d'angle d'irradiation d'au moins 180°, mais de moins 360°, à un second volume partiel,
c) reconstruction de l'absorption du voxel dans le premier volume partiel eu égard aux données de mesure de toutes les positions de sources de rayons,
d) reconstruction de l'absorption du voxel dans le second volume partiel eu égard aux données de mesure filtrées de précisément autant de positions de sources de rayons qu'il n'en faut pour obtenir un domaine d'angle d'irradiation de 180°.

5. Appareil de tomographie assistée par ordinateur agencé pour exécuter la procédé suivant la revendication 1, comportant une source de rayons et une unité de détecteurs couplée à la source ainsi qu'un dispositif d'entraînement pour faire tourner un objet contenu dans la zone d'examen et la source de rayons l'un par rapport à l'autre autour d'un axe de rotation et une unité de reconstruction pour la reconstruction de la répartition spatiale de l'absorption à l'intérieur de la zone d'examen à partir des données de mesure acquises par l'unité de détecteurs,
**caractérisé par**
a) des moyens pour réarranger les données de mesure en plusieurs groupes, chaque groupe comportant plusieurs plans parallèles à l'axe de rotation, dans lesquels se trouve chaque fois un éventail de rayons,
b) des moyens pour le filtrage unidimensionnel des données produites par le réarrangement de chaque groupe dans une direction perpendiculaire à la direction des plans
c) des moyens pour la reconstruction de la répartition spatiale de l'absorption à partir des données filtrées de différents groupes.

6. Appareil de tomographie assistée par ordinateur suivant la revendication 5, comportant un dispositif de collimation façonné d'une manière telle que le faisceau de rayons conique soit ouvert parallèlement à l'axe de rotation de telle sorte que l'angle d'ouverture correspondant au milieu du faisceau de rayons conique soit plus petit qu'aux bords de ce faisceau.
